Europäisches Patentamt

European Patent Office

Office européen des brevets

(19)

(11) Numéro de publication: **0 188 931**
**B1**

(12) **FASCICULE DE BREVET EUROPEEN**

(45) Date de publication du fascicule du brevet:
16.08.89

(51) Int. Cl.⁴: **C 12 N 9/02,** C 12 N 1/14 //
(C12N1/14, C12R1:645)

(21) Numéro de dépôt: 85402377.7

(22) Date de dépôt: 03.12.85

(54) Microorganismes de souche Phanerochaete chrysosporium et leur utilisation.

(30) Priorité: **12.12.84 FR 8419028**

(43) Date de publication de la demande:
30.07.86 Bulletin 86/31

(45) Mention de la délivrance du brevet:
16.08.89 Bulletin 89/33

(84) Etats contractants désignés:
AT BE CH DE FR GB IT LI LU NL SE

(56) Documents cités:
FEMS MICROBIOLOGY LETTERS, vol. 25, 1984, pages 295-299, Elsevier, Federation of European Microbiological Societies; J.A. BUSWELL et al: "Ligninolytic enzyme production by Phanerochaete chrysosporium under conditions of nitrogen sufficiency"
PROC. NATL. ACAD. SCI. USA, vol. 81, avril 1984, pages 2280-2284; M. TIEN et al.: "Lignin-degrading enzyme from Phanerochaete chrysosporium: Purification, characterization, and catalytic properties of a unique H2O2-requiring oxygenase"
CHEMICAL ABSTRACTS, vol. 98, no. 18, 2 mai 1983, page 103, résumé no. 145235p, Columbus, Ohio, US; I.D. REID: "Effects of nitrogen supplements on degradation of aspen wood lignin and carbohydrate components by Phanerochaete chrysosporium" & APPL. ENVIRON.

(73) Titulaire: **INSTITUT NATIONAL DE LA RECHERCHE AGRONOMIQUE, Etablissement public dit:, 145, Rue de l'Université, F-75341 Paris Cédex 07 (FR)**

(72) Inventeur: **Buswell, John Anthony, 24, Lomond Crescent, Beith, Aryshire KA15 2EA Ecosse (GB)**
Inventeur: **Odier, Etienne, 98, rue Saint Antoine, F-75004 - Paris (FR)**

(74) Mandataire: **Gillard, Marie-Louise et al, Cabinet Beau de Loménie 55, Rue d'Amsterdam, F-75008 Paris (FR)**

(56) Documents cités: (suite)
MICROBIOL. 1983, 45(3), 830-7
CHEMICAL ABSTRACTS, vol. 98, no. 18, 2 mai 1983, page 104, résumé no. 145250q, Columbus, Ohio, US; I.D. REID: "Effects of nitrogen sources on cellulose and synthetic lignin degradation by Phanerochaete chrysosporium" & APPL. ENVIRON. MICROBIOL. 1983, 45(3), 838-42

ACTORUM AG

## Description

La présente invention concerne de nouveaux microorganismes de souche Phanerochaete chrysosporium ainsi que leur utilisation pour la production de l'enzyme ligninolytique.

La biodégradation de la lignine joue un rôle essentiel dans le cycle du carbone terrestre.

Ce composé, après la cellulose, représente le matériau organique le plus abondant capable de se renouveler. Il a en outre la faculté de s'incruster et d'empêcher, jusqu'au moment de sa décomposition, l'accès des bactéries dégradantes vers la cellulose et les hémicelluloses des tissus des plantes ligneuses.

Les champignons Basidiomycètes qui provoquent la pourriture blanche du bois sont capables de dégrader tous les constituants de la paroi végétale, y compris la lignine. La dégradation de ce polymère phénolique a été plus particulièrement étudiée chez le champignon Phanerochaete chrysosporium.

Ce champignon et d'autres espèces causant la pourriture blanche en question sont par conséquent susceptibles d'applications dans des domaines divers tels que la technologie du bois et du papier et la production de composés chimiques à partir de déchets lignocellulosiques.

Néanmoins, on pourrait élargir l'éventail de telles applications potentielles si l'on disposait de méthodes permettant la sélection de souches de capacités supérieures.

Différentes études sur la régulation nutritionnelle de la dégradation de la lignine par le champignon Phanerochaete chrysosporium ont établi une relation nette entre une carence en azote du milieu de culture et l'apparition d'une activité ligninolytique.

Par exemple, P. chrysosporium ME-446 (ATCC 34 541) ne dégrade pas la lignine pendant sa phase de croissance active, mais pendant la phase stationnaire, lorsqu'elle se trouve en carence nutritionnelle.

La dégradation de la lignine par ce champignon représente par conséquent une caractéristique d'un métabolisme secondaire, l'influence de l'azote jouant un rôle dans l'établissement et le maintient de cette activité ligninolytique.

Aussi, KEYSER et coll. ont montré dans J. of Bacteriology, sept. 1978, pp. 790–797 que la décomposition de la lignine par P. chrysosporium s'effectue de manière significative uniquement lorsque le milieu de culture est carencé en azote.

L'intensité de la répression du système ligninolytique par l'azote varie selon la source d'azote choisie. L'acide glutamique est un puissant répresseur de la ligninolyse et parait jouer, avec la glutamine, un rôle privilégié dans la régulation de l'activité ligninolytique [FENN et coll., Arch. Microbiol. 130, pp. 59–65 (1981)].

Chez d'autres microorganismes, un métabolisme secondaire est déclenché sous l'influence de diverses carences nutritionnelles.

Dans le cas de P. chrysosporium, le système ligninolytique peut être stimulé également sous l'influence d'une carence du milieu de culture en hydrate de carbone et en soufre bien que seule une carence en azote ait été jugée nécessaire à l'obtention d'une dégradation importante de la lignine (JEFFRIES et coll., Applied and Environmental Microbiology, Aug. 1981, pp. 290–296).

MING TIEN et T. KENT KIRK ont rapporté dans Science 221, pp. 661–663 (1983), la découverte d'une enzyme extracellulaire produite par Phanerochaete chrysosporium Burdsall, enzyme capable de provoquer, en présence de peroxyde d'hydrogène, la dégradation oxydante de différents composés-modèles ayant la sous-structure de la lignine de même que la dégradation de la lignine de sapin ou de bouleau.

Cette enzyme produite par P. chrysosporium BKM-1767 (ATCC 24 725) ou par P. chrysosporium ME-446 (ATCC 34 541) a été décrite dans Proc. Natl. Acad. Sci. U.S.A., vol. 81, pp. 2280–2284 (1984) comme étant une glycoprotéine d'environ 42 000 daltons contenant un protoheme IX par molécule. Elle catalyse de manière non stéréospécifique plusieurs oxydations dans les chaînes alkyles latérales de modèles moléculaires de type lignine (monomères ou dimères) ou de lignines macromoléculaires: rupture $C_\alpha$–$C_\beta$ dans les composés du type aryl-$C_\alpha$–HOH–$C_\beta$HR–$C_\gamma$H$_2$O (R=aryle ou O-aryle), oxydation des alcools benzyliques en aldéhydes ou cétones, rupture intradiol des structures phénylglycol et hydroxylation du radical méthylène des groupements benzyliques. L'enzyme en question catalyse également le couplage oxydatif des phénols expliquant probablement la relation connue depuis longtemps entre l'oxydation phénolique et la dégradation de la lignine.

Toutes ces réactions nécessitent la présence de peroxyde d'hydrogène pour leur accomplissement.

Cette enzyme constitue une oxygénase unique due au fait qu'elle nécessite le peroxyde d'hydrogène pour exercer son activité.

Pour des raisons de commodité, l'enzyme ainsi décrite sera dénommée par la suite «enzyme ligninolytique».

Les conditions de production de cette enzyme sont cependant encore mal maîtrisées et la lenteur du processus de biodégradation de la lignine constitue un facteur limitant pour les applications actuellement envisagées: délignification enzymatique ou microbienne de sous-produits agricoles ou forestiers, en vue de l'amélioration de leur digestibilité pour les ruminants, de l'hydrolyse enzymatique de la cellulose, ou d'une fermentation anaérobie.

Par conséquent, l'obtention de souches de P. chrysosporium hyperligninolytiques capables de déréguler le système ligninolytique serait d'un atout primordial pour de futures applications industrielles.

On a maintenant trouvé, de manière surprenante, que des microorganismes de souche Phanerochaete chrysosporium sont capables, dans des milieux non limités en azote, de développer une activité ligninolytique beaucoup plus élevée que les souches P. chrysosporium connues, par conséquent, selon des conditions opératoires plus avantageuses que celles exigées par l'art antérieur.

Ainsi, un premier objet de l'invention concerne deux nouveaux microorganismes de l'espèce Phanerochaete chrysosporium Burdsall.

Un autre objet de l'invention concerne l'utilisation des nouveaux microorganismes Phanerochaete chrysosporium selon l'invention pour la production de l'enzyme ligninolytique.

Les microorganismes selon l'invention sont déposés auprès de l'INSTITUT PASTEUR (Paris) dans la Collection Nationale de Cultures de Microorganismes (C.N.C.M.) sous les numéros respectifs I-398 et I-399.

Un autre objet de l'invention vise en outre un procédé pour l'obtention d'un surnageant de culture contenant l'enzyme ligninolytique, procédé suivant lequel on cultive les nouveaux microorganismes Phanerochaete chrysosporium de l'invention en milieu nutritif non carencé en source d'azote assimilable et contenant une source de carbone et de sels minéraux assimilables.

De même, l'invention se rapporte à un procédé pour l'obtention de l'enzyme ligninolytique, procédé suivant lequel on cultive les nouveaux microorganismes Phanerochaete chrysosporium de l'invention en milieu nutritif non carencé en source d'azote assimilable et contenant une source de carbone et de sels minéraux assimilables, pour obtenir un surnageant de culture contenant l'enzyme ligninolytique que l'on isole par exemple au moyen de techniques de centrifugation, dialyse et chromatographie.

Suivant un mode de réalisation pour l'obtention d'un surnageant contenant l'enzyme ligninolytique en question, on prépare un inoculum végétatif en ensemençant une petite quantité du milieu de culture avec une suspension conidiale du microorganisme.

On cultive alors ce microorganisme sous conditions d'aérobiose ou de préférence sous conditions d'oxygène, à une température comprise entre 28 °C et 40 °C de préférence à 37 °C et sur différents milieux nutritifs contenant une source d'azote, une source de carbone et des sels minéraux.

Avant l'inoculation par le microorganisme, il est souhaitable d'ajuster le pH du milieu de culture à 4,5-5. On utilise, à cet effet un tampon diméthyl-2,2' succinate de sodium ajusté avec un hydroxyde de métal alcalin par exemple l'hydroxyde de potassium.

En général, une production significative d'enzyme ligninolytique au sein du surnageant a lieu du 3ème au 8ème jour avec un maximum au 5ème ou 6ème jour.

Comme source de carbone assimilable, on utilise par exemple le glucose, le mannose, l'amidon, le melibiose, le mannitol, le xylose, le maltose, l'adonitol, l'arabitol, le fructose, le sorbitol, le raffinose, le xylitol, le D(+)treholose ou le glycérol.

La source d'azote assimilable peut être par exemple l'asparagine, le nitrate d'ammonium ou le tartrate d'ammonium.

Quant aux sels minéraux ceux-ci peuvent être par exemple le citrate de fer, $KH_2PO_4$, $ZnSO_4$, $MnSO_4$, $CaCl_2$, $CuSO_4$, $NaCl$, $FeSO_4$, $CoCO_4$, $ZnSO_4$, $AIK(SO_4)_2$, $H_3BO_3$, $Na_2MoO_4$, $MgSO_4$.

Le glycérol constitue la source de carbone préférée selon l'invention.

On a remarqué, en effet, que cette source nutritive en milieu non carencé en azote permet d'obtenir des taux extrêmement importants d'activité ligninase ou même ligninolytique au contraire des souches P. chrysosporium BKM-1767 (ATCC 24725) et ME-446 (ATCC 34 541) qui ne produisent pas d'enzyme ligninolytique dans ces conditions.

Comme rapporté précédemment, une carence en azote et en carbone du milieu de culture représente une condition nécessaire à l'apparition d'une activité ligninolytique significative chez toutes les souches de P. chrysosporium examinées jusqu'à présent.

Ainsi, la culture des microorganismes, selon l'invention, dans des milieux non carencés en azote ou en glycérol permettrait d'obtenir l'activité ligninolytique en question de manière continue, ce qui ne pourrait être envisageable avec les souches de P. chrysosporium connues puisqu'il est nécessaire, dans ce cas, d'ajuster continuellement le milieu de culture pour respecter les carences nutritionnelles.

L'enzyme ligninolytique contenue dans les surnageants des cultures des microorganismes de l'invention peut être isolée notamment par centrifugation, dialyse et chromatographie par exemple par chromatographie sur colonne de gel d'aragose.

L'enzyme ainsi obtenue peut être alors conservée après lyophilisation.

Des cultures des nouveaux microorganismes ont été réalisées de manière à étudier l'activité enzymatique ou ligninase, l'activité ligninolytique ainsi que divers paramètres tels que l'influence des sources de carbone et d'azote sur la croissance du mycélium ou sur l'activité enzymatique.

I. Croissance du mycélium

On a utilisé, à cet effet, le processus général décrit dans l'Exemple ci-après en faisant varier la qualité de la source de carbone et/ou la teneur en azote (asparagine/$NH_4NO_3$) du milieu nutritif et on a déterminé les poids secs des mycélia, après isolement et séchage, au moyen de filtres en fibre de verre de 2,5 mm de diamètre.

Les résultats obtenus avec P. chrysosporium C.N.C.M. -I-398 apparaissent dans le dessin en annexe sur lequel:

– Fig. 1: la courbe (1) représente l'effet sur la croissance du mycélium, d'une carence en azote du milieu de culture (2,6 mM) en présence de glucose comme source de carbone
– Fig. 2: la courbe (1) représente l'effet, sur la croissance du mycélium, d'une haute teneur en azote du milieu de culture (26 mM) en présence de glucose comme source de carbone

- Fig. 3: la courbe (1) représente l'effet, sur la croissance du mycélium, d'une haute teneur en azote du milieu de culture (26 mM) en présence d'amidon comme source de carbone

- Fig. 4: la courbe (1) représente l'effet, sur la croissance du mycélium, d'une haute teneur en azote du milieu de culture (26 mM) en présence de glycérol comme source de carbone.

Les résultats montrent notamment que le glycérol apparaît comme une source de carbone peu favorable à la croissance de P. chrysosporium C.N.C.M. -I-398.

Sur glucose, comme source de carbone, que le milieu soit limité ou pas en azote, la croissance du mycélium est nettement plus importante.

A titre de comparaison, on a déterminé la croissance de P.chrysosporium C.N.C.M. -I-398 et d'une souche connue à savoir P. chrysosporium BKM-1767 (ATCC 24 725) en milieu de culture à haute teneur en azote (26 mM), le glycérol étant la source de carbone (pH initial du milieu: 4,5):

| Durée de l'incubation (j) | P. chrysosporium BKM-1767 | | P. chrysosporium C.N.C.M.-I-398 | |
|---|---|---|---|---|
| | Poids du mycélium sec (mg) | pH | Poids du mycélium sec (mg) | pH |
| 2 | 15,8 ± 1,6 | 5,46 ± 0,04 | 4,9 ± 0,3 | 5,37 ± 0,04 |
| 3 | 34,2 ± 2,6 | 4,27 ± 0,05 | 10,0 ± 1,0 | 5,05 ± 0,04 |
| 4 | 39,2 ± 3,1 | 4,08 ± 0,04 | 11,3 ± 1,5 | 5,34 ± 0,02 |
| 5 | 40,2 ± 0,7 | 4,06 ± 0,01 | 16,6 ± 0,4 | 5,33 ± 0,06 |
| 6 | 37,7 ± 1,3 | 4,17 ± 0,09 | 19,9 ± 1,0 | 4,76 ± 0,18 |
| 7 | 37,8 ± 1,3 | 4,46 ± 0,06 | 20,8 ± 0,4 | 4,45 ± 0,08 |

Ces résultats montrent que dans les conditions de l'essai la croissance de la souche connue est beaucoup plus importante que celle de P. chrysosporium C.N.C.M.-I-398.

Des essais ultérieurs ont permis de constater que les conditions d'oxygénation n'influencent pas la croissance de P. chrysosporium C.N.C.M.-I-398 comme l'indiquent les résultats ci-dessous obtenus à partir d'un milieu contenant le glycérol comme source de carbone et caractérisé par une haute teneur en azote:

| Temps d'incubation (j) | Cultures mises sous atmosphère de 100% d'oxygène après inoculation | | Cultures mises sous atmosphère d'air après inoculation | |
|---|---|---|---|---|
| | Poids sec du mycélium (mg) | pH | Poids sec du mycélium (mg) | pH |
| 2 | 4,5 ± 1,1 | 5,22 ± 0,00 | 4,5 ± 0,2 | 5,10 ± 0,04 |
| 3 | 11,1 ± 1,3 | 5,23 ± 0,08 | 11,8 ± 3,2 | 4,97 ± 0,13 |
| 4 | 16,2 ± 2,5 | 4,97 ± 0,14 | 15,3 ± 0,9 | 5,14 ± 0,01 |
| 5 | 18,5 ± 2,4 | 5,12 ± 0,08 | 16,9 ± 3,1 | 5,11 ± 0,06 |
| 6 | 22,5 ± 4,0 | 4,35 ± 0,09 | 18,0 ± 2,5 | 5,20 ± 0,09 |
| 7 | 25,2 ± 0,5 | 4,38 ± 0,02 | 22,2 ± 1,5 | 5,14 ± 0,07 |

Au cours de ces essais, on a également remarqué que la souche P. chrysosporium BKM-1767 (ATCC 24 725) présente une sporulation après 5 jours devenant intense après 6 jours.

Dans le cas de P. chrysosporium C.N.C.M.-I-398 on a observé une consistance hydrophobe du mycélium et une sporulation intense après 5 jours.

Toutefois, chez P. chrysosporium I-399 aucune sporulation n'a été remarquée.

II. Activité ligninase
A. Sur substrat pour l'enzyme ligninolytique

On a déterminé l'activité ligninase de surnageants de cultures obtenus tel que décrit dans l'Exemple ci-après en mesurant à 35 °C l'augmentation de l'absorbance à 310 nm due à l'oxydation de l'alcool vératrylique (ou diméthoxy-3,4 benzylique) en vératraldéhyde, selon la méthode décrite dans Proc. Natl. Acad. Sci. U.S.A., Vol. 81, pp. 2280–2284 (1984).

A cet effet, on a employé le même mélange réactionnel que celui de la référence en question à l'exception du peroxyde d'hydrogène que l'on a utilisé à raison de 0,27 mM.

On a défini l'activité enzymatique en unités, une unité correspondant à 1 nmole/min. de vératraldéhyde formé dans les conditions de l'essai.

Les résultats obtenus avec P. chrysosporium C.N.C.M.-I-398 sont repris dans le dessin en annexe sur lequel:

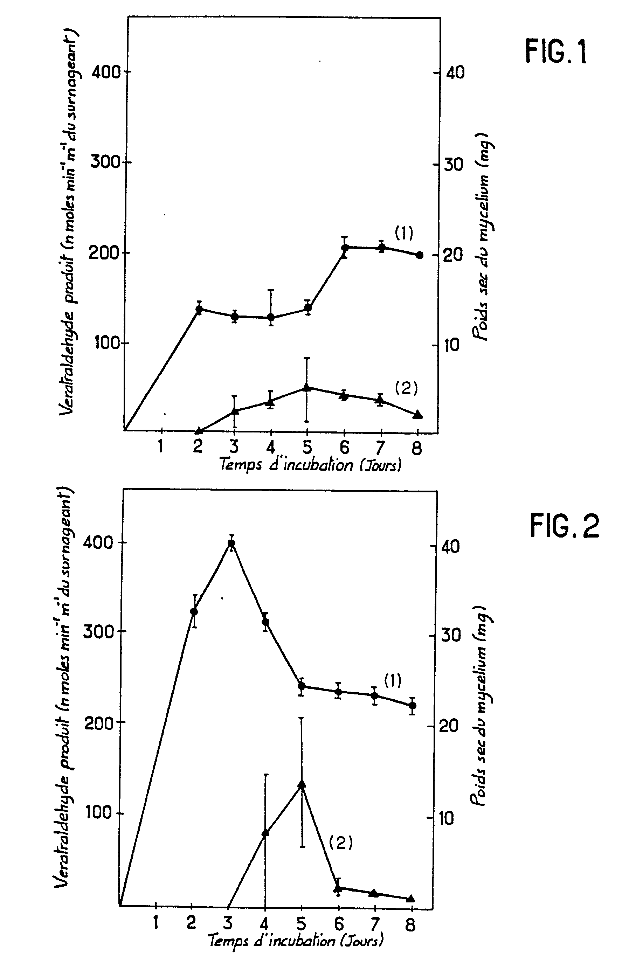

– Fig. 1: la courbe (2) représente l'effet, sur l'activité ligninase d'une carence en azote du milieu de culture (2,6 mM) en présence de glucose comme source de carbone

– Fig. 2: la courbe (2) représente l'effet, sur l'activité ligninase d'une haute teneur en azote du milieu de culture (26 mM) en présence de glucose comme source de carbone

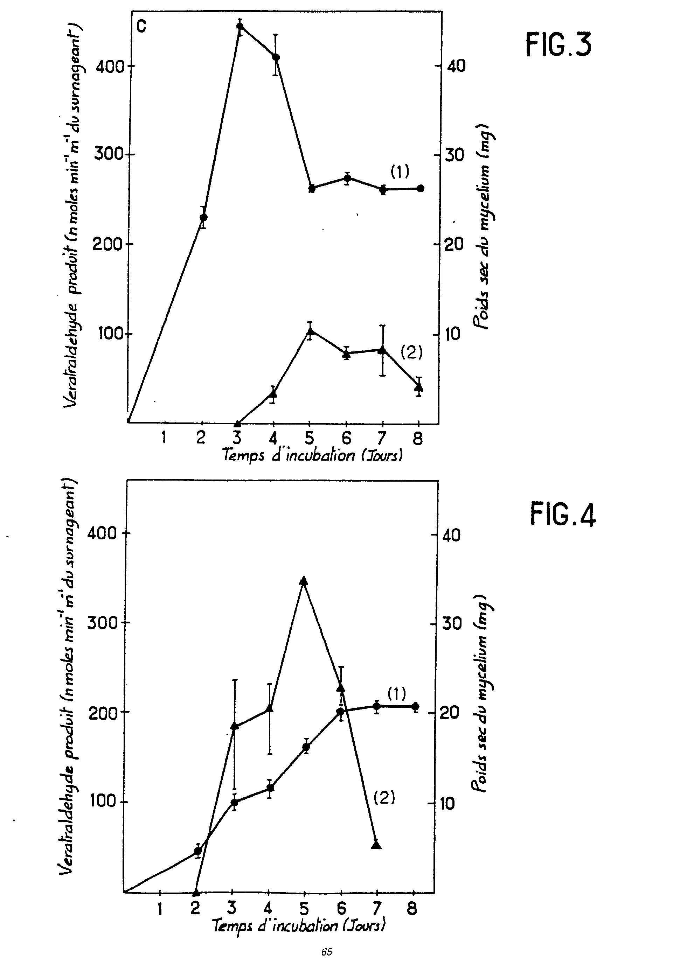

– Fig. 3: la courbe (2) représente l'effet, sur l'activité ligninase d'une haute teneur en azote du milieu de culture (26 mM) en présence d'amidon comme source de carbone

– Fig. 4: la courbe (2) représente l'effet, sur l'activité ligninase d'une haute teneur en azote du milieu de culture (26 mM) en présence de glycérol comme source de carbone.

La Fig. 1 montre que lorsque P. chrysosporium C.N.C.M.-I-398 est cultivé selon des conditions de l'état de la technique, c'est-à-dire en milieu carencé en azote et contenant le glucose comme source de carbone, une activité ligninase apparaît dans les surnageants des cultures après 3 jours.

Cependant la valeur maximale de cette activité, de l'ordre de 50 unités après 5 jours, se révèle faible et comparable à l'activité enzymatique décelée dans des cultures comparatives d'une souche connue à savoir P. chrysosporium ME-446 (ATCC 34 541) (10 à 20 unités).

Par contre, les Fig. 2 et 3 montrent qu'une activité ligninase est aisément détectable dans des cultures à haute teneur en azote (26 mM) et dans lesquelles le glucose ou l'amidon soluble, constituent la source de carbone.

Dans les cultures à haute teneur en azote et contenant soit le glucose soit l'amidon comme source de carbone, l'activité est encore significative 4 jours après que la croissance du mycélium ait atteint un maximum.

Dans les cultures à haute teneur en azote utilisant le glycérol comme source de carbone, on constate que la production enzymatique est très largement augmentée.

L'activité apparaît entre 2 et 3 jours après l'inoculation et dépasse souvent 350 unités après 5 jours.

Les taux élevés d'activité enzymatique observés lorsque le glycérol constitue la source de carbone sont très probablement en relation avec le faible taux de croissance de P. chrysosporium C.N.C.M.-I-398 sur le glycérol comparativement au glucose.

A titre de comparaison, on a déterminé l'activité ligninase obtenue par culture de P. chrysosporium C.N.C.M.-I-398 et d'une souche connue à savoir P. chrysosporium BKM-1767 (ATCC 24 725) en milieu de culture à haute teneur en azote (26 mM) et en glycérol.

Les résultats obtenus sont les suivants:

| Durée de l'incubation (j) | P. chrysosporium BKM-1767 | P. chrysosporium C.N.C.M.-I-398 |
|---|---|---|
| | Unités d'activité ligninase/ml du milieu | |
| 2 | 0 | 0 |
| 3 | 11 ± 9 | 182 ± 69 |
| 4 | 0 | 203 ± 42 |
| 5 | 2 ± 2 | 353 ± 0 |
| 6 | 3 ± 4 | 224 ± 40 |
| 7 | 1 ± 1 | 58 ± 4 |

Ces résultats prouvent à nouveau que l'activité ligninase est produite par P. chrysosporium C.N.C.M.-I-398 moyennant une haute teneur en azote du milieu de culture au contraire de la souche P. chrysosporium connue.

Des essais complémentaires effectués dans les mêmes conditions c'est-à-dire en milieu glycérol non carencé en azote ont révélé des taux d'activité ligninase de 490,7 ± 4,5 unités et 641,8 ± 61,6 unités par culture respectivement de P. chrysosporium C.N.C.M.-I-398 et P. chrysosporium C.N.C.M.-I-399.

B. Sur composés-modèles de lignine

On a déterminé l'activité enzymatique de surnageants de cultures obtenus tel que décrit dans l'Exemple ci-après.

A cet effet, on a mesuré, à 35 °C, la formation du triméthoxy-3,4,5 benzaldéhyde dû à la dégradation oxydative, dépendante du peroxyde d'hydrogène, du (triméthoxy-3,4,5 phényl)-1 (diméthoxy-2,6 hydroxyméthyl-4 phényl)-2 propanediol-1,3 selon la méthode décrite dans Proc. Natl. Acad. Sci. U.S.A., Vol. 81, pp. 2280–2284 (1984) pour les dimères β-1 (diarylpropane) non radioactifs.

De même, on a mesuré, à 35 °C, la formation du vératraldéhyde dû à la dégradation oxydative, dépendante du peroxyde d'hydrogène, du (diméthoxy-3,4 phényl)-1 (méthoxy-2 phénoxy)-2 propanediol-1,3 selon la méthode décrite également dans Proc. Natl. Acad. Sci., U.S.A., Vol. 81, pp. 2280–2284 (1984) pour les dimères β-0-4 éthers (arylglycérol-aryléther).

Les résultats de ces essais ont révélé un taux important d'activité ligninase mise en évidence par la rupture des liaisons $C_\alpha$–$C_\beta$ des composés-modèles utilisés.

On a également pu mettre en évidence un effet important de la pression partielle de l'oxygène sur des milieux de culture de P. chrysosporium C.N.C.M. I-398 contenant le glucose comme source de carbone mais un effet moins marqué sur des milieux contenant le glycérol.

Les cultures de ce microorganisme, en milieu glucose non carencé en azote, nécessitent absolument une atmosphère d'oxygène pur pour l'apparition d'une activité ligninase alors que les cultures du même microorganisme en milieu glycérol non carencé en azote développent cette activité en atmosphère d'air ou d'oxygène.

Cependant, les taux d'activité enzymatique relevés en atmosphère d'air se sont révélés quatre fois plus faibles qu'en atmosphère d'oxygène pur.

III. Activité ligninolytique

On a réalisé les tests d'activité ligninolytique suivants en utilisant comme lignine synthétique le $^{14}C$–DHP (DHP: polymère par déshydrogénation d'alcool coniférylique).

1) On décante des surnageants obtenus après 5 jours de culture de P. chrysosporium C.N.C.M.-I-398 tel que décrit dans l'Exemple ci-après. On les combine et on réintroduit 9 ml du mélange obtenu dans les flacons contenant les cultures.

On ajoute alors 0,5 ml d'une solution à 20% de glucose (poids/vol) ainsi que 0,5 unité de glucose oxydase (0,5 ml d'une solution fraîchement préparée dans l'eau distillée) de manière à stimuler la réaction. On introduit 0,1 ml d'une suspension de $^{14}C$–DHP (5480 dpm), on provoque alors un balayage continuel des flacons par l'oxygène et on piège le $^{14}CO_2$ formé dans 4 ml d'un mélange phénéthylamine/méthanol/eau (2:1:1). On ajoute ensuite 10 ml d'un liquide de scintillation et on mesure la radioactivité dans un compteur à scintillation.

On a obtenu les résultats suivants exprimés en % du $^{14}C$ total disponible:

| Temps (h) après initiation de la réaction | % $^{14}C$ initial cumulé (converti en $^{14}CO_2$) |
|---|---|
| 2,5 | 54 ± 4 |
| 5 | 65 ± 7 |
| 24 | 66 ± 7 |

Ces résultats montrent que des milieux de culture de P. chrysosporium-I-398 permettent de provoquer un taux important de dégradation de la lignine synthétique en présence de peroxyde d'hydrogène généré par le système glucose/glucose oxydase.

Dans d'autres essais semblables, on a obtenu jusqu'à 79% du $^{14}C$ sous forme de $^{14}CO_2$ après 2 heures.

2) On réalise à 37 °C des cultures de P. chrysosporium C.N.C.M.-I-398 en flacon de 250 ml équipé de manière à permettre un balayage par l'oxygène.

On utilise, pour ces cultures des milieux semblables à celui décrit dans l'Exemple ci-après (glucose/2,6 mM en azote et glycérol/26 mM en azote) dans lesquels on ajoute 0,1 ml d'une suspension de $^{14}C$–DHP.

On inocule les cultures avec approximativement 2,3·10⁵ spores, on balaie périodiquement avec de l'oxygène à 100% pendant 2 min. et on piège le $^{14}CO_2$ formé dans 4 ml d'un mélange phénéthylamine/méthanol/eau (2:1:1). On ajoute 10 ml d'un liquide de scintillation et on mesure la radioactivité dans un compteur à scintillation.

On a obtenu les résultats suivants exprimés en % du $^{14}C$ total disponible:

| Durée de l'incubation (j) | % $^{14}C$ initial cumulé (converti en $^{14}CO_2$) | |
|---|---|---|
| | glucose/2,6 mM en azote | glycérol/26 mM en azote |
| 7 | 11 | 47 |
| 8 | 15 | 48 |
| 9 | 17 | 49 |
| 12 | 23 | 50 |
| 14 | 25 | 52 |

Ces résultats confirment la supériorité des conditions de culture préférées selon l'invention (haute teneur en glycérol et azote) sur la dégradation de la lignine synthétique en essai prolongé.

L'Exemple non limitatif suivant illustre l'invention.

Exemple
Préparation de l'enzyme ligninolytique
a) Surnageant de culture contenant l'enzyme ligninolytique

On maintient P. chrysosporium C.N.C.M.-I-398 à 4 °C sur des plaques d'agar à 2% de malt.

D'autre part, on prépare un inoculum, consistant en suspensions conidiales filtrées sur laine de verre, en utilisant le milieu de sporulation décrit dans Applied and Environmental Microbiology, Vol. 35, No. 6 pp. 1223–1225 (1978).

On réalise ensuite des cultures (1% de glycérol et 26 mM en azote) dans des flacons Erlenmeyer de 150 ml contenant 10 ml du milieu aqueux de composition suivante:

| Source de carbone | mg |
|---|---|
| Glycérol | 100 |
| Source d'azote | |
| Asparagine | 10 |
| $NH_4NO_3$ | 5 |
| Sels minéraux | |
| $KH_2PO_4$ | 2 |
| $MgSO_4, 7H_2O$ | 0,5 |
| $CaCl_2, 2H_2O$ | 0,132 |
| $MnSO_4, 4H_2O$ | 0,05 |
| Citrate de fer | 0,12 |
| $ZnSO_4, 7H_2O$ | 0,066 |
| $CuSO_4, 5H_2O$ | 0,001 |
| $CoCl_2, 6H_2O$ | 0,001 |
| Vitamine | |
| Thiamine | 0,025 |
| Tampon | |
| Diméthyl-2,2' succinate de sodium | 14,6 |

et on ajuste à pH 4,5–5 avec de l'hydroxyde de potassium.

On inocule les cultures avec approximativement 2,3 · 10⁵ spores, on balaie avec de l'oxygène pendant 2 min. après inoculation et on bouche les flacons hermétiquement.

On maintient alors les cultures à 37 °C et on obtient 3 à 4 jours après inoculation un surnageant contenant l'enzyme ligninolytique, l'activité enzymatique maximale se situant vers 5 à 6 jours.

b) Séparation de l'enzyme ligninolytique

On combine les cultures de 6 jours et on centrifuge à 4 °C pendant 15 min. (10 000 g). On ajoute au surnageant du fluorure de p-méthylsulfonyle (0,2 mM) pour réduire la protéolyse et on concentre sur filtre (dimension des pores: 10 000 daltons) jusqu'à 250 ml. On dialyse durant 8 à 10 heures contre une solution tampon 5 mM en tartrate de sodium (pH=4,5) et on introduit dans une colonne de chromatographie contenant du gel d'agarose équilibrée au préalable avec la même solution tampon.

On lave la colonne avec 100 ml de la solution tampon et on introduit un gradient salin (0–0,1 M de chlorure de sodium dans une solution à 5 mM en tartrate de sodium, pH=4,5, volume total: 400 ml).

Toutes les étapes de purification se déroulent à 4 °C. On dialyse alors la solution enzymatique contre de l'eau distillée désionisée et on garde à −20 °C l'enzyme ligninolytique sous forme de poudre lyophilisée stable.

En utilisant la méthode ci-dessus maisen remplaçant le glycérol par le glucose ou l'amidon, on a obtenu également l'enzyme ligninolytique.

De même, on a pu produire l'enzyme en question en remplaçant P. chrysosporium C.N.C.M.-I-398 par P. chrysosporium C.N.C.M.-I-399.

## Revendications pour les etats contractants: BE, CH, DE, FR, GB, IT, LI, LU, NL, SE

1. Microorganisme de souche Phanerochaete chrysosporium Burdsall déposé sous le No. C.N.C.M.-I-398.

2. Microorganisme de souche Phanerochaete chrysosporium Burdsall déposé sous le No. C.N.C.M.-I-399.

3. Microorganisme selon la Revendication 1 ou 2 pour la production d'un surnageant de culture contenant l'enzyme ligninolytique.

4. Microorganisme selon la Revendication 1 ou 2 pour la production de l'enzyme ligninolytique.

5. Procédé pour la production d'un surnageant de culture contenant l'enzyme ligninolytique caractérisé en ce que l'on cultive, à une température comprise entre 28 et 40 °C, un microorganisme selon la Revendication 1 ou 2 en milieu nutritif non carencé en source d'azote assimilable et contenant une source de carbone et de sels minéraux assimilables.

6. Procédé pour la production de l'enzyme ligninolytique caractérisé en ce que l'on cultive, à une température comprise entre 28 et 40 °C, un microorganisme selon la Revendication 1 ou 2 en milieu nutritif non carencé en source d'azote assimilable et contenant une source de carbone et de sels minéraux assimilables pour obtenir un surnageant de culture contenant l'enzyme ligninolytique, enzyme que l'on isole par centrifugation, dialyse et chromatographie.

7. Procédé selon la Revendication 5 ou 6 caractérisé en ce que la source de carbone est le glycérol.

8. Procédé selon la Revendication 5 ou 6 caractérisé en ce que la source d'azote est l'asparagine et/ou le nitrate d'ammonium.

## Revendications pour l'Etat contractant: AT

1. Procédé pour l'obtention de l'enzyme ligninolytique caractérisé en ce que l'on cultive, à température comprise entre 28 et 40 °C, en milieu nutritif non carencé en source d'azote assimilable et contenant une source de carbone et de sels minéraux assimilables, un microorganisme de souche Phanerochaete chrysosporium Burdsall choisi parmi les microorganismes déposé sous les numéros I-398 et I-399 à la Collection Nationale de Cultures de Microorganismes (C.N.C.M.) pour obtenir un surnageant de culture contenant ladite enzyme ligninolytique et en ce qu'éventuellement on isole par centrifugation, dialyse et chromatographie l'enzyme ligninolytique.

2. Procédé selon la revendication 1, caractérisé en ce que la source de carbone est le glycérol.

3. Procédé selon la revendication 1, caractérisé en ce que la source d'azote est l'asparagine et/ou le nitrate d'ammonium.

## Patentansprüche für die Vertragsstaaten: BE, CH, DE, FR, GB, IT, LI, LU, NL, SE

1. Mikroorganismus des Stamms Phanerochaete chrysosporium Burdsall, hinterlegt unter der No. CNCM-I-398.

2. Mikroorganismus des Stamms Phanerochaete chrysosporium Burdsall, hinterlegt unter der No. CNCM-I-399.

3. Mikroorganismus nach Anspruch 1 oder 2 zur Herstellung eines Kulturüberstandes, der das Ligninolyseenzym enthält.

4. Mikroorganismus nach Anspruch 1 oder 2 zur Herstellung des Ligninolyseenzyms.

5. Verfahren zur Herstellung eines Kulturüberstandes, der das Ligninolyseenzym enthält, dadurch gekennzeichnet, dass man einen Mikroorganismus nach Anspruch 1 oder 2 in einem Nährmedium, das keinen Mangel an einer assimilierbaren Stickstoffquelle aufweist und eine Quelle an assimilierbarem Kohlenstoff und assimilerbaren Mineralsalzen enthält, bei einer Temperatur zwischen 28 und 40 °C kultiviert.

6. Verfahren zur Herstellung des Ligninolyseenzyms, dadurch gekennzeichnet, dass man einen Mikroorganismus nach Anspruch 1 oder 2 in einem Nährmedium, das keinen Mangel an einer assimilierbaren Stickstoffquelle aufweist und eine Quelle an assimilierbarem Kohlenstoff und assimilierbaren Mineralsalzen enthält, bei einer Temperatur zwischen 28 und 40 °C kultiviert, um einen Kulturüberstand zu erhalten, der das Ligninolyseenzym enthält, welches Enzym man durch Zentrifugieren, Dialyse und Chromatografie isoliert.

7. Verfahren nach Anspruch 5 oder 6, dadurch gekennzeichnet, dass die Kohlenstoffquelle Glyzerin ist.

8. Verfahren nach Anspruch 5 oder 6, dadurch gekennzeichnet, dass die Stickstoffquelle Asparagin und/oder Ammoniumnitrat ist.

**Patentansprüche für den Vertragsstaat: AT**

1. Verfahren zur Herstellung des Ligninolyseenzyms, dadurch gekennzeichnet, dass man einen Mikroorganismus des Stamms Phanerochaete chrysosporium Burdsall, ausgewählt aus den unter den Nummern I-398 und I-399 bei der Collection Nationale de Cultures de Microorganismes (CNCM) [Nationale Sammlung von Kulturen von Mikroorganismen] hinterlegten Mikroorganismen, in einem Nährmedium, das nicht an einer assimilierbaren Stickstoffquelle mangelt und eine Quelle an assimilierbarem Kohlenstoff und assimilierbaren Mineralsalzen enthält, bei einer Temperatur zwischen 28 und 40 °C kultiviert, um einen Kulturüberstand zu erhalten, der das Ligninolyseenzym enthält, und man gegebenenfalls das Ligninolyseenzym durch Zentrifugieren, Dialyse und Chromatografie isoliert.

2. Verfahren nach Anspruch 1, dadurch gekennzeichnet, dass die Kohlenstoffquelle Glyzerin ist.

3. Verfahren nach Anspruch 1, dadurch gekennzeichnet, dass die Stickstoffquelle Asparagin und/oder Ammoniumnitrat ist.

**Claims for Contracting States: BE, CH, DE, FR, BG, IT, LI, LU, NL, SE**

1. A microorganism of the Phanerochaete Chrysosporium Burdsall strain, deposited under No. C.N.C.M.-I-398.

2. A microorganism of the Phanerochaete Chrysosporium Burdsall strain, deposited under No. C.N.C.M.-I-399.

3. A microorganism according to claim 1 or 2 for the production of a culture supernatant containing lignin-degrading enzyme.

4. A microorganism according to claim 1 or 2 for the production of lignin-degrading enzyme.

5. A process for the production of a culture supernatant containing lignin-degrading enzyme, characterized in that it comprises cultivating a micro-organism according to claim 1 or 2, at a temperature of between 28 and 40 °C, in a nutrient medium which is not deficient in a source of assimilable nitrogen and which contains a source of assimilable carbon and assimilable mineral salts.

6. A process for the production of lignin-degrading enzyme, characterized in that it comprises cultivating a microorganism according to claim 1 or 2, at a temperature of between 28 and 40 °C, in a nutrient medium which is not deficient in a source of assimilable nitrogen and which contains a source of assimilable carbon and assimilable mineral salts, to give a culture supernatant containing lignin-degrading enzyme, this enzyme being isolated by centrifugation, dialysis and chromatography.

7. The process according to claim 5 or 6, characterized in that the carbon source is glycerol.

8. The process according to claim 5 or 6, characterized in that the nitrogen source is asparagine and/or ammonium nitrate.

**Claims for Contracting State: AT**

1. A process for the production of lignin-degrading enzyme, characterized in that it comprises cultivating, at a temperature of between 28 and 40 °C, in a nutrient medium which is not deficient in a source of assimilable nitrogen and which contains a source of assimilable carbon and assimilable mineral salts, a microorganism of the Phanerochaete chrysosporium Burdsall strain chosen from the microorganismes deposited under the numbers I-398 and I-399 in the Collection Nationale de Cultures de Microorganismes (C.N.C.M.), to give a culture supernatant containing the said lignin-degrading enzyme, and, if appropriate, isolating the lignin-degrading enzyme by centrifugation, dialysis and chromatography.

2. The process according to claim 1, characterized in that the carbon source is glycerol.

3. The process according to claim 1, characterized in that the nitrogen source is asparagine and/or ammonium nitrate.

## 1/2

FIG.1

FIG.2

FIG.3

FIG.4